# EUROPEAN PATENT APPLICATION

(11) **EP 3 053 536 A1**
(43) Date of publication of application: **10.08.2016**
(21) Application number: 15200079.0
(22) Date of filing: 15.12.2015
(51) Int. Cl.: A61B 18/12, A61B 18/14

(54) **ELECTRONIC DEVICE FOR DIATHERMY WITH ACTIVE CONTROL OF CURRENT**

(30) Priority: 19.12.2014 IT NA20140041 U
(71) Applicant: Brera Medical Technologies S.r.l., 84061 Ogliastro Cilento (SA) (IT)
(72) Inventor: GRATTACASO, Attilio, 84061 Ogliastro Cilento (SA) (IT)
(74) Representative: Conversano, Gabriele

(57) **Abstract**

Electronic device for carrying out diathermy, comprising a first and a second electrode, configured to be positioned on the body of a patient and to apply a time-variable voltage thereto, a control device, electrically connected to said electrodes, characterized in that said control device comprises means for measuring the electric current supplied by said electrodes and control means for varying the voltage applied to said electrodes, as a function of the quantity of current supplied.

## Description

The present invention relates to a device for carrying out diathermy, a therapy which exploits heat as tool for treating, in a not superficial manner, body parts affected by chronic pathologies, characterized by degeneration and fibrosis.

Such therapies provide the passage of currents through the body, to which they are applied by means of electrodes. Therefore, by the Joule effect it is generated heat which, by causing an increase in temperature of the treated area, re-vascularizes the fibrous scar tissue formed after the biological damage, thus restoring the correct metabolism.

At the state of the art, there are known many equipments which, by exploiting such principle, allow to realize this treatment in various areas of the body. Generally, these electro-medical apparatuses work by positioning two electrodes, generally by means of adhesive plates, on the area of the body to be treated; in the following, a current of intensity selected on the basis of the pathology of the patient is circulated by means of these electrodes through the body of the patient. This current, by the Joule effect, generates a certain quantity of heat which is a function of the body impedance, in turn dependent on the distance to which the electrodes are positioned on the area of the body to be treated. Such heat produces an increase in temperature in the area of the body to be treated where the electrodes are positioned. But the increase in temperature is not superficial, as it would occur by heating a body part in a traditional manner, but it is internal.

The intensity of current, and so the temperature reached, are selected on the basis of the pathology to be treated. In case of using electrodes positioned on adhesive plates, it is easily to be realized that the position of such plates is fixed during the treatment, and so with such electrodes only the area of the body can be treated locally, where they are positioned.

At the state of the art, there are also known equipments which use a first fixed electrode (of the adhesive type), which is generally positioned at little distance to the area needing the diathermic treatment, and a second mobile electrode, which is moved by means of a suitable handle which allows a movement thereof similar to the one of a massage, for treating wider areas of the body. These apparatuses are always more used, since it is a dynamic treatment which allows to treat wider areas without the need of moving the electrodes each time, and giving the patient the pleasant feeling of a massage at the same time.

The problem occurred while using this kind of equipments relates exactly the quantity of heat generated inside the body of the patient, which is variable in the moving away and approaching step between the electrodes, and changes also according to the body parts on which the electrode moves. In fact, since the heat is generated by the Joule effect, its entity will depend on the impedance which the current finds between the two electrodes, when passing through the body; the impedance is a function of the distance between the two electrodes but it depends also on the particular tissue of the body between an area of the body or another one. Therefore, during the movement of the electrode, while the impedance changes, the quantity of heat generated changes as well and so does the temperature reached; this, in some cases, can generate burns in not superficial areas of the body where the impedance is low, in others it can cause a mild effect in areas where the impedance has instead a high value. As it is known, in fact, the dissipated electric current is proportional to the squared voltage applied and inversely proportional to the impedance.

To obviate this problem, at the state of the art there are known solutions which use an impedance meter, i.e. an equipment which measures the body impedance. But also this solution has some drawbacks: in fact, if variable voltages with frequencies up to about 100 kHz are used, it is obtained a reliable measuring of the body impedance, and so the treatment can be carried out suitably. When, instead, such frequency is exceeded, the value of the body impedance provided by the impedance meter is no more valid, since the body impedance, beyond that threshold, has no more a constant value but it depends on the frequency. As a consequence it is not possible to use frequencies beyond the threshold of 100 kHz. Anyway, it is known that to increase the frequencies allows to work in safety and without damaging the inner tissues, since the curve of the action potential (which represents the excitability of the cells of the human body by current impulses) is such that while the applied frequency increases, the intensity of the stimulus needed to produce the excitation of the cell increases: while the frequency increases, the duration of the stimulus is such short that the current does not influence the status of the cell. This is the reason why there is the need to work with high frequencies in the application of diathermy. eter is no more valid, since the body impedance, beyond that threshold, has no more a constant value but it depends on the frequency. As a consequence it is not possible to use frequencies beyond the threshold of 100 kHz. Anyway, it is known that to increase the frequencies allows to work in safety and without damaging the inner tissues, since the curve of the action potential (which represents the excitability of the cells of the human body by current impulses) is such that while the applied frequency increases, the intensity of the stimulus needed to produce the excitation of the cell increases: while the frequency increases, the duration of the stimulus is such short that the current does not influence the status of the cell. This is the reason why there is the need to work with high frequencies in the application of diathermy.

Therefore, aim of the present invention is to provide a device for carrying out diathermy by means of variable voltages with frequency higher than 100 kHz, by keeping the control of current and the respective heat generated during the movement of the electrode.

In particular, the present invention provides an electronic device for carrying out diathermy, provided with active control of current, which allows to keep constant the thermal effect while varying the body impedance of the patient. According to another aim the present invention provides a device for carrying out diathermy comprising a handgrip provided with a constraining means for constraining removably interchangeable electrodes, and configured so that the electric features of the positioned electrode are recognized.

The invention solves the aims since it is an electronic device for carrying out diathermy, comprising: a first (11) and a second electrode (3), configured to be positioned on the body of a patient and to apply a time-variable voltage thereto; a control device (9), electrically connected to said electrodes (11, 3), characterized in that said control device (9) comprises means for measuring the electric current supplied by said electrodes (3, 11) and control means for varying the voltage applied to said electrodes (3, 11) as a function of the quantity of current supplied.

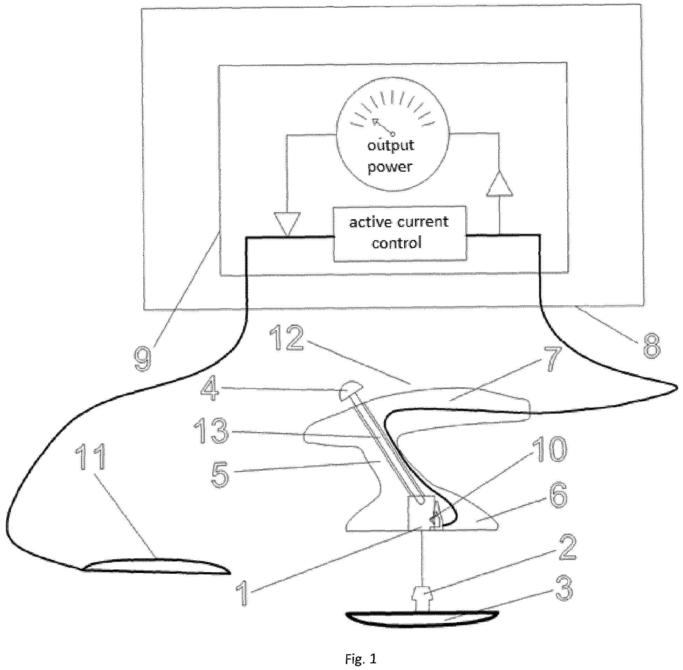

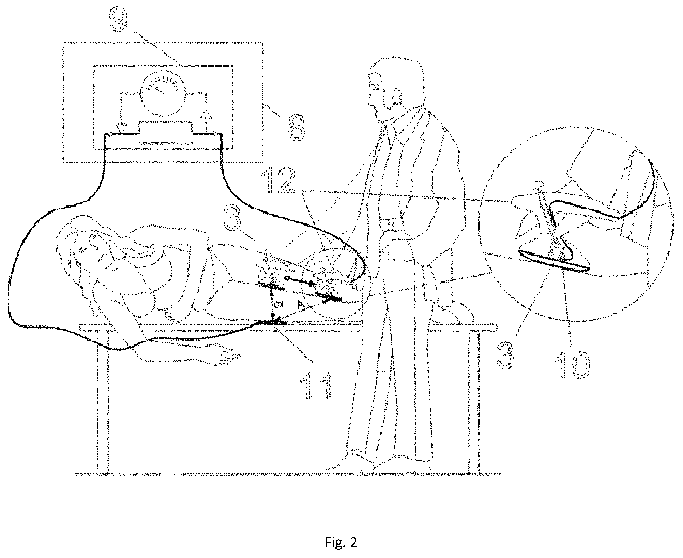

These and other advantages will be clear from the detailed description of the invention, which will refer to the appended drawings. In figure 1 it is shown a schematic view of the electronic device for carrying out the diathermy, according to the present invention and its respective handgrip; in figure 2 it is shown the use of the device according to the present invention.

With reference to figure 1, the device according to the present invention comprises a first (11) and a second electrode (3), both being electrically connected to a control device (9) contained inside a suitable case (8).

Conveniently, the control device (9) is a logic board with microprocessor (9) configured to read continuously the current passing through the body of the patient by means of said electrodes (11, 3). Said control device (9) is also configured to vary continuously the voltage applied between the first electrode (11) and the second electrode (3), as a function of the reading of current carried out and so that the dissipated power is kept constant at a level defined by the operator.

Moreover, conveniently said second electrode (3) comprises a "snap" projection, also said hook (2), configured to engage a suitable housing (1) provided in the lower part of the handgrip (12) (which faces the patient during functioning). Said housing (1) is configured to house said projection (2) and to hold it by means of a spring constraining means (10). The constraint between the electrode (3) and the spring constraining means (10) is configured to be disengaged by controlling a button (4), positioned on the upper surface of said handgrip and configured to push by means of a rod (13) on said projection (2), thus expelling said second electrode (3).

The handgrip (12) is configured for making easier for the operator to grasp it, so that the operator can easily move the electrode on the patient, as it is shown in figure 2.

The handgrip (12) is preferably provided with a gripping handle (7) having at the end a shaft (5) positioned approximately orthogonally to the grip handle (7) and ending with a spherical cap (6), in the lower portion of which it is provided said housing (1).

The control device (9) can be set to supply a determined electric power between the first electrode (11) and the second electrode (3). Once the value of the electric power to be supplied is set, the control device adjusts the value of the variable voltage applied as a function of the reading of the current passing through the body of the patient, measured constantly by said control device. In this way, it is possible to keep constant the thermal effect in the body of the patient during the movement of the electrode (3) on the parts of the body of the patient to be treated with diathermy.

In fact, while varying the position of the electrode (3) the distance between the two electrodes will change and, as a consequence, the impedance of the body. If the voltage applied were not changed, there would be an excessive or very weak supplied power. The variation of the voltage applied as a function of the intensity of current measured instead allows to control the electric power supplied.

In the view of figure 2, it is represented an operator who moves the handgrip (12) to which the second electrode (3) is constrained, by moving it away and approaching it to the first electrode (11) positioned under the leg of the patient. In the imagine, it can be noted how the distance between the second electrode (3) and the first electrode (11) changes while it moves, passing from a distance "B" to a greater distance "A"; the body impedance will change while the distance between the first electrode (3) and the second electrode (11) changes; in the view it is represented how the logic board with microprocessor (9) reads continuously the current passing through the body of the patient by means of the first electrode (11) and the second electrode (3), varying continuously the electric voltage applied between said first (11) and second electrode (3), so that the value of the supplied electric power is kept constant.

In addition, preferably the control device is configured to detect automatically the features of the second electrode (3) interchangeable on the universal handgrip (12), thus determining if the same is of capacitive or resistive type, by means of a measuring of the current passing through the first electrode (11) and the second electrode (3).

## Claims

1. Electronic device for carrying out diathermy, comprising:
a first (11) and a second electrode (3),
configured to be positioned on the body of a patient and to apply a time-variable voltage thereto;
a control device (9), electrically connected to
said electrodes (11, 3),
**characterized in that**
said control device (9) comprises means for measuring the electric current supplied by said electrodes (3, 11) and control means for varying the voltage applied to said electrodes (3, 11) as a function of the quantity of current supplied.

2. Device according to claim 1, **characterized in that** said control device is configured so that said applied voltage is changed so that the supplied power is kept constant, equal to a value set by the operator.

3. Device according to claim 1 or 2, further comprising a handgrip (12) provided with a housing (1) to constrain removably said second electrode (3).

4. Device according to claim 3, **characterized in that** said constraint occurs by means of a spring constraining means (10) configured to engage a "snap" projection (2) provided on said electrode, and **in that** said handgrip (12) comprises also a button (4) positioned on the higher surface of said handgrip (12) and configured to push by means of a rod (13) on said projection (2), thus expelling said second electrode (3).

5. Device according to any one of the preceding claims, **characterized in that** said second electrode (3) can be of resistive or capacitive type and **in that** said control device is configured to detect the type of said second electrode by measuring the electric current.
